# EUROPÄISCHE PATENTSCHRIFT

(11) **EP 1 372 490 B1**
(45) Veröffentlichungstag und Bekanntmachung des Hinweises auf die Patenterteilung: **01.09.2004**
(21) Anmeldenummer: 02727458.8
(22) Anmeldetag: 25.03.2002
(51) Int. Cl.: A61B 17/04

(54) **CHIRURGISCHE NÄHMASCHINE**
SURGICAL SUTURING MACHINE
MACHINE A SUTURE CHIRURGICALE

(30) Priorität: 26.03.2001 DE 10116171
(43) Veröffentlichungstag der Anmeldung: 02.01.2004
(73) Patentinhaber: Karl Storz GmbH & Co., 78532 Tuttlingen (DE)
(72) Erfinder: MOLL,Philip, 52078 Aachen (DE); KLUNDT, Kurt, 67732 Hirschhorn (DE)
(74) Vertreter: Klein, Friedrich
(86) Internationale Anmeldenummer: PCT/EP2002/003242
(87) Internationale Veröffentlichungsnummer: WO 2002/076304

(56) Entgegenhaltungen:
- US-A- 2 580 964
- US-A- 4 747 358
- US-A- 5 496 334

## Beschreibung

Die Erfindung betrifft eine chirurgische Nähmaschine, insbesondere eine Nähmaschine zur Herstellung von Nähten innerhalb des Körpers von Menschen oder Tieren.
Nähmaschinen dieser Art sollen, da sie in den menschlichen oder tierischen Körper eingeführt werden müssen, ein möglichst geringes Bauvolumen aufweisen, weshalb nicht nur die eigentlichen Bedienelemente sondern möglichst auch die Antriebe für die Stichbildewerkzeuge in einem außerhalb des Körpers verbleibenden Gehäuse angeordnet sind und mittels Übertragungsmitteln mit diesen verbunden sind.

Die US 4 841 888 zeigt eine als "endoskopische Nähmaschine" bezeichnete Nähmaschine mit einem rohrformigen Gehäuse gemäss dem Oberbegriff des Anspruchs 1, an dessen in den Körper einzuführendem Ende die Stichbildewerkzeuge angeordnet sind, und dessen freies anderes Ende die Bedienelemente trägt, die über innerhalb des rohrförmigen Gehäuses angeordnete Übertragungsmittel mit den Stichbildewerkzeugen verbunden sind.

Da mit dieser Nähmaschine nur ein Einfaden-Kettenstich der Stichtype 101 (DIN 5300 Teil 1) erzeugt werden soll, sind die Stichbildewerkzeuge und auch deren Bewegungen relativ einfach und werden von einer im rohrförmigen Gehäuse längsverschiebbar angeordneten, einen Faden führenden Nadel und einem mit dieser zusammenwirkenden Greifer gebildet.
Der Greifer ist dabei von einem im wesentlichen V-förmig gestalteten Hebel gebildet, der am Ende des einen Schenkels als Greiferspitze ausgebildet ist. Das Ende des anderen Schenkels ist an einem elastischen Ansatz des Gehäuses schwenkbar gelagert, der gleichzeitig als Auflagefläche für die Greiferspitze dient. Sobald die Nadel während ihrer durch ein drahtförmig ausgebildetes Betätigungselement eingeleitete Schlingenhubbewegung ausgeführt hat, wird der Greifer durch ein an der Spitze des "V" angreifendes, ebenfalls drahtförmig ausgebildetes zweites Betätigungselement in die gebildete Fadenschleife geschwenkt. Am Ende dieser Schwenkbewegung liegt die Greiferspitze auf dem Ansatz auf, sodaß die vom Greifer erfaßte Fadenschlinge bis zum nächsten Einstich der Nadel durch den Greifer gehalten wird und die Nadel in das von der Fadenschlinge gebildete Fadendreieck zum Abstich derselben einstechen kann.

Da sowohl zur Erzeugung der Längsbewegungen der Nadel, als auch zur Erzielung der Schwenkbewegungen des Greifers jeweils ein drahtförmig ausgebildetes Betätigungselement völlig ausreichend ist, ist der konstruktive Aufwand hierfür relativ gering.

Nachteilig ist jedoch, daß hierdurch nur ein einfacher Kettenstich der Stichtype 101 gebildet wird und hierdurch nur eine einfache Geradnaht hergestellt werden kann, die insbesondere auch im medizinischen Bereich gegenüber einer sogenannten Überdecknaht nachteilig ist.

Der Erfindung liegt daher die Aufgabe zugrunde, eine für den Einsatz als endoskopische Nähmaschine geeignete Nähmaschine zu schaffen, die es ermöglicht, eine Überdecknaht unter Verwendung einer dem Stichtyp 501 entsprechenden Stichart zu schaffen.

Diese Aufgabe wird durch eine Nähmaschine gelöst, die ein Gehäuse aufweist, bestehend aus einem Gehäuseoberteil zur Aufnahme von Antrieben für die Stichbildewerkzeuge, einem sich an dieses anschließenden Gehäuseschaft zur Aufnahme von Mitteln zur Übertragung der von den Antrieben erzeugten Bewegungen auf die Stichbildewerkzeuge die mindestens eine fadenführende Nadel und einen mit dieser zusammenwirkenden Greifer aufweisen, der nach dem Erfassen der durch die Nadel gebildeten Fadenschlinge entlang einer mehrdimensionalen Bewegungsbahn von seiner unterhalb des Nähgutes gelegenen, die Fadenschlinge erfassenden Position in eine oberhalb des Nähgutes gelegene Position bewegbar ist, in der das von der auf die Nähgutoberseite geführten Fadenschlinge gebildete Fadendreieck die Projektion der Nadelbahn umschließt.

Da der Greifer bei der erfindungsgemäßen Lösung sowohl die von der Nadel gebildete Fadenschlinge aufnimmt, als auch diese von der unterhalb des Nähgutes gelegenen Erfassungsposition zunächst in eine seitlich des Nähgutes gelegene Zwischenlage und von dort in eine oberhalb der Ebene des Nähgutes gelegene Position bringt, um sie dann zum Abstechen durch die Nadel in einer Lage zu positionieren, in welcher die Bewegungsbahn der Nadel durch das von der Fadenschlinge gebildete Fadendreieck verläuft, kann an Stelle des Zwei-Greifer-Systems ein einziger Greifer verwendet werden, sodaß nicht nur der zweite Greifer, sondern auch die entsprechenden Antriebsmittel für diesen entfallen, wobei sich durch die mehrdimensionale Bewegungsbahn des Greifers zusätzlich der Raumbedarf für den Antrieb des Greifers erheblich reduziert und der Greifer von nur einem einzigen Antriebsmittel, nämlich von der einzigen Greiferwelle, angetrieben werden kann.

In konstruktiver Hinsicht läßt sich die hierfür erforderliche Bewegungsbahn des Greifers dann relativ einfach realisieren, wenn diese aus einer unterhalb des Nähgutes erfolgenden ersten Schwingbewegung, einer sich hieran anschließenden Hubbewegung und einer zweiten Schwingbewegung zusammengesetzt ist.

Eine besonders raumsparende Anordnung ergibt sich dann, wenn die den Greifer tragende Greiferwelle im Gehäuse längsverschiebbar und drehbar angeordnet ist und ihr ein Schwingantrieb und ein Hubantrieb zugeordnet ist, deren Antriebsmittel (Kurvenscheiben) auf ein und derselben Welle angeordnet sind, die parallel zur Greiferwelle gerichtet ist.

Dabei ist es vorteilhaft wenn einerseits das Abtriebsteil (Träger 44) des Hubantriebes in axialer Richtung mit der Greiferwelle starr verbunden ist und diese im Abtriebsteil (Träger 44) frei drehbar ist und andererseits das Abtriebsteil (Buchse 59) des Schwingantriebes (60) mit der Greiferwelle (39) drehfest verbunden ist und diese in axialer Richtung im Abtriebsteil (Buchse 59) längsverschieblich ist.

Um die vorerwähnte Doppelfunktion des Greifers zu realisieren, ist es erforderlich, daß der seitliche Abstand der die Fadenschlinge erfassenden Hauptklinge zur Nadel während des Erfassens der Fadenschlinge wesentlich geringer ist, als deren seitlicher Abstand zur Nadel während des Positionierens der erfaßten Fadenschlinge oberhalb des Nähgutes zum Einstich der Nadel in das von der Fadenschlinge gebildete Fadendreieck. Dies bedeutet, daß -in Blickrichtung vom Rand des Nähgutes auf die Nadel- die Hauptklinge des Greifers sich um einen bestimmten Betrag hinter der Bewegungsbahn der Nadel befinden muß, da Gewähr für einen Einstich der Nadel in das Fadendreieck nur dann gegeben ist, wenn sich die Nadel auf einer Bahn bewegt, die einen bestimmten Abstand zur kurzen Seite des von der Fadenschlinge gebildeten Dreiecks hat.

Diese unterschiedliche Position der Hauptklinge in ihrer jeweiligen Endstellung läßt sich dadurch erreichen, wenn die zum Erfassen der Fadenschlinge dienende erste Schwingbewegung des Greifers um eine Achse erfolgt, deren Abstand zur Nadel größer ist, als der Abstand der Nadel zu der Achse, um die die zur Positionierung der Fadenschlinge auf dem Nähgut dienende zweite Schwingbewegung erfolgt.

Dies läßt sich in konsruktiv einfacher und raumsparender Weise dann realisieren, wenn die Greiferwelle von einem Oberteil und einem Unterteil gebildet ist und beide mittels eines Gelenkstückes derart miteinander verbunden sind, daß das Unterteil in horizontaler Richtung relativ zum Oberteil bewegbar ist.
Die zum Erfassen der Fadenschlinge erforderliche Schwenkbewegung kann dann bei in der einen Richtung ausgeschwenktem Unterteil der Greiferwelle erfolgen, während die zur Positionierung der Fadenschlinge oberhalb des Nähgutes erforderliche Schwenkbewegung bei in der anderen Richtung ausgeschwenktem Unterteil der Greiferwelle erfolgen kann, d.h wenn sich das Unterteil der Greiferwelle in horizontaler, parallel zum Rand des Nähgutes verlaufender Richtung um einen bestimmten Betrag zur Nadel hin bewegt hat.

Zur Erzielung der für die unterschiedlichen Auslenkungen des Unterteils der Greiferwelle erforderlichen Relativbewegung zwischen dem Oberteil und dem Unterteil ist es zweckmäßig einen Horizontalantrieb vorzusehen, der eine von einer Kurvenscheibe abgeleitete Hubbewegung einer Schubstange bewirkt, die über eine Antriebsverbindung als Horizontalbewegung auf das Unterteil übertragbar ist.

Eine insgesamt raumsparende Anordnung wird dann erzielt, wenn die Kurvenscheibe für den Horizontalantrieb auf einer Welle angeordnet ist, die zu der die Kurvenscheiben von Schwing- und Hubantrieb aufnehmenden Welle parallel gerichtet ist, und oberhalb der Kurvenscheiben für den Schwing- und Hubantrieb eine weitere Kurvenscheibe angeordnet ist, die zum Antrieb eines Fadenlenkers dient.

Eine besonders raumsparende Anordnung ergibt sich dann die Kurvenscheibe für den Lenkertrieb eine Stange betätigt, die innerhalb der Schubstange längsverschieblich angeordnet ist, wobei deren aus der Schubstange hervorstehendes Ende dem Fadenlenker über eine Antriebsverbindung eine aus einer vertikalen und einer horizontalen Bewegungskomponente gebildete Bewegung erteilt. Durch die koaxiale Anordnung der Schubstange für den Horizontalantrieb für das Unterteil der Greiferwelle und der Stange für den Antrieb des Fadenlenkers ergibt sich ein besonders kleiner Querschnitt für den Gehäuseschaft.

Um während der oberhalb des Nähgutes erfolgenden Schwenkbewegung der Hauptklinge des Greifers von ihrer die Fadenschlinge haltenden Position in die für die Ausführung ihrer Senkbewegung erforderliche Stellung eine Kollision zwischen der Hauptklinge und der Fadenschlinge zu vermeiden, weist der Fadenlenker eine im wesentlichen V-förmige, schräg nach aufwärts gerichtete Aufnahmenut für den oberen Schenkel der Fadenschlinge auf, deren Schräglage zum leichten Erfassen des Schenkels der Fadenschlinge zum Zeitpunkt der Aufnahme des Schenkels der Fadenschlinge im wesentlichen parallel zu dieser verläuft. Durch die dabei dem Fadenlenker gleichzeitig erteilbare horizontale Bewegungskomponente wird die Fadenschlinge zusätzlich aus der Bewegungsbahn der Hauptklinge gebracht.

Zur Sicherung der von der Hauptklinge des Greifers erfaßten Fadenschlinge während deren Schwenkbewegung weist der Greifer eine Gegenklinge auf, deren Spitze zur Spitze der Hauptklinge im wesentlichen entgegengesetzt gerichtet ist.

Weitere Einzelheiten und Vorteile ergeben sich aus den Unteransprüchen und der nachfolgenden Beschreibung einer in der beigefügten Zeichnung dargestellten bevorzugten Ausführungsform der Erfindung.

Es zeigt:
**Fig. 1:** eine schematische Gesamtansicht einer endoskopischen Nähmaschine;
**Fig. 1a:** eine vergrößerte Darstellung einer Einzelheit "A" der Fig.1;
**Fig. 2**: eine schaubildliche Darstellung des Griffteils und des Mittelteils des Gehäuses, teilweise geschnitten;
**Fig. 3**: eine schaubildliche Darstellung des Hub- und Schwingantriebes sowie einen Teil des Horizontalantiebes für die Greiferwelle;

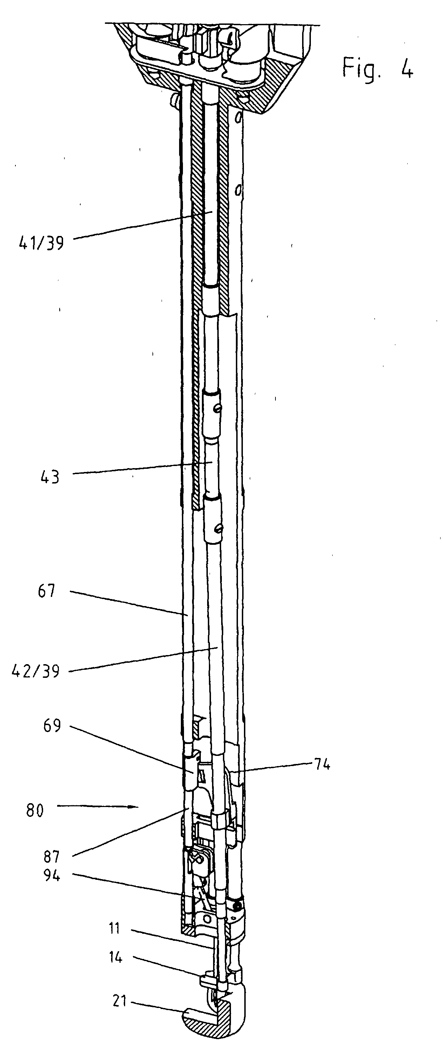

**Fig. 4:** eine Darstellung des Gehäuseschaft 3 mit entferntem Schutzrohr;
**Fig. 5:** eine vergrößerte Darstellung des unteren Bereiches des Gehäuseschaftes;
**Fig. 5a:** eine vergrößerte Darstellung einer Einzelheit des Horizontalantriebes 80 für den Greifer 12;
**Fig. 5b:** eine vergrößerte Darstellung einer Einzelheit des Lenkertriebes für den Fadenlenker 13;
**Fig. 6a:** eine Draufsicht des Greifers 12;
**Fig. 6b:** eine Seitenansicht des Greifers 12;
**Fig. 6c:** eine vergrößerte perspektifische Darstellung des Greifers 12;
**Fig. 7:** eine Darstellung der Hebe- und Senkeinrichtung für den Niederhalter 14;
**Fig. 7a:** eine vergrößerte Darstellung einer Einzelheit "B" der Fig.7;
**Fig. 7b:** eine vergrößerte Darstellung einer Einzelheit "C" der Fig.7;
**Fig. 8:** eine schaubildliche Darstellung von Nadel, Greifer, Niederhalter und Fadenlenker mit dem Fadenverlauf mit sich im Bereich ihrer unteren Totpunktlage befindlicher Nadel vor dem Schlingenhub;
**Fig. 9:** eine der Fig. 8 entsprechende Darstellung mit sich seitlich des Nähgutes befindlichem Greifer vor seiner Aufwärtsbewegung;
**Fig. 10:** eine der Fig. 8 entsprechende Darstellung mit sich im Bereich ihrer oberen Totpunktlage befindlicher Nadel;
**Fig. 11:** eine der Fig. 8 entsprechende Darstellung mit sich ca 20 bis 40° nach ihrer oberen Totpunktlage befindlicher Nadel;
**Fig. 12:** eine der Fig. 8 entsprechende Darstellung mit sich ca 120° nach ihrer oberen Totpunktlage befindlicher Nadel;

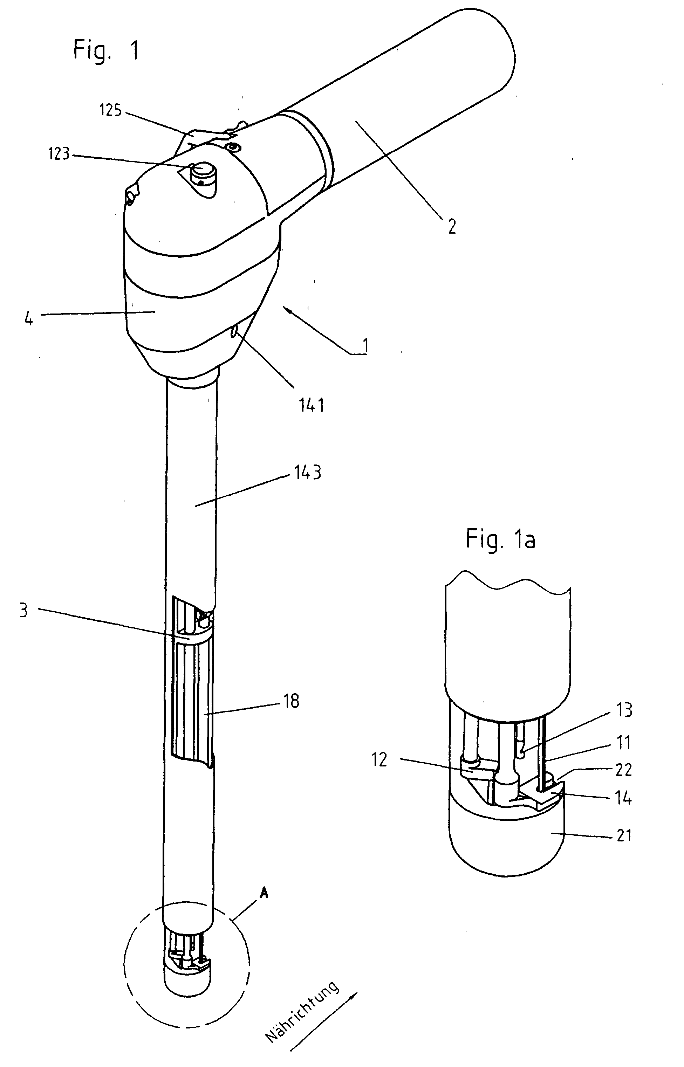

Die in Fig. 1 schematisch dargestellte endoskopische Nähmaschine 1 weist ein im wesentlichen L-förmiges Gehäuse auf, das von einem als Hohlkörper ausgebildeten Griffteil 2, einem zu diesem im wesentlichen senkrecht gerichteten Gehäuseschaft 3 und einem diesen mit dem Griffteil 2 verbindenden Gehäusemittelteil 4 gebildet ist.

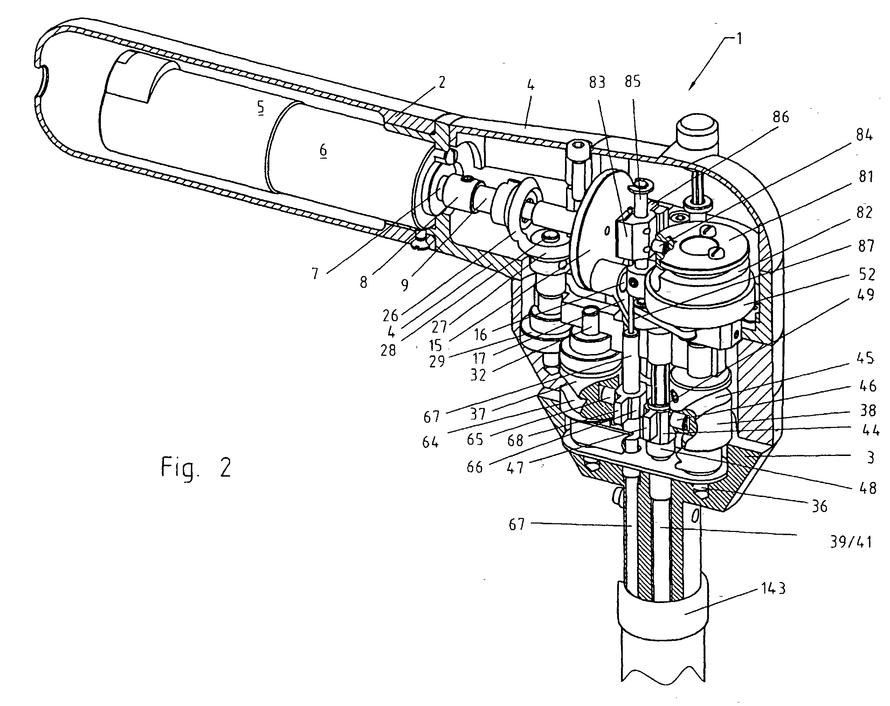

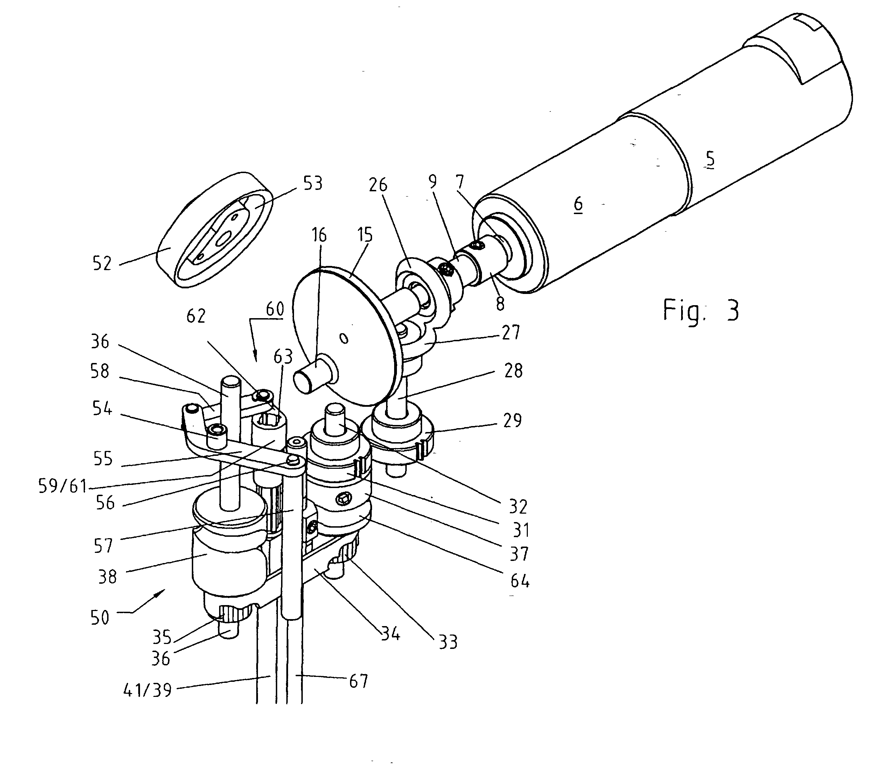

Innerhalb des Griffteiles 2 ist (Fig.2 und 3) ein Elektromotor angeordnet, der vorzugsweise als Gleichstrommotor 5 ausgebildet ist. Die nicht dargestellte Abtriebswelle des Gleichstrommotors 5 ist mit einer ebenfalls nicht dargestellten Eingangswelle eines vorzugsweise als Planetengetriebe ausgebildeten und innerhalb des Griffteils 2 angeordneten Untersetzungsgetriebes 6 starr verbunden, dessen Abtriebswelle 7 mittels einer in an sich bekannter Weise ausgebildeten lösbaren Kupplung 8 mit einer Hauptwelle 9 verbunden ist. Die Hauptwelle 9, von der die Bewegungen für eine Nadel 11 und einen Greifer 12, sowie für einen mit diesem zusammenwirkenden Fadenlenker 13 und einen Niederhalter 14 abgeleitet sind, ist im Gehäusemittelteil 4 fliegend gelagert und trägt an ihrem freien Ende eine Kurbelscheibe 15, an deren Zapfen 16 ein Lenker 17 angreift, der seinerseits an eine die Nadel 11 tragende Nadelstange 18 angelenkt ist. Die vorbeschriebenen Teile bilden einen Nadelantrieb 20.

Zur optischen Erfassung der Drehzahl und/oder des Drehwinkels der Hauptwelle 9 können an der Kurbelscheibe 15 parallel zu ihrer Längsachse gerichtete Bohrungen oder sonstige Markierungen vorgesehen sein. Die Verbindung zwischen dem Lenker 17 und der Nadelstange 18 kann in an sich bekannter Weise lösbar ausgebildet sein, um hierdurch die Lage des unteren oder oberen Umkehrpunktes der Bewegung der Nadel 11 einstellen zu können.

Die Nadelstange 18 ist in am Gehäuseschaft 3 vorgesehenen Führungen in Richtung ihrer Längsachse geführt und führt vertikal gerichtete Hubbewegungen aus, wobei die Spitze der fadenführenden Nadel 11 durch ein an einer Stichplatte 21 vorgesehenes schlitzförmig ausgebildetes Stichloch 22 hindurchtritt. Die Stichplatte 21 ist im Bereich des unteren Endes des Gehäuseschaftes 3 an diesem ausgebildet, wobei ihr seitlicher Rand 23 in bestimmtem seitlichem Abstand zum Stichloch 22 verläuft.
Beim gezeigten Ausführungsbeispiel ist die Nadelstange 18 aus baulichen Gründen etwas seitlich zur Hauptwelle 9 versetzt angeordnet.

Da mit der erfindungsgemäßen Nähmaschine eine Kettenstichnaht des Stichtypes 501 oder eines ähnlichen Stichtypes hergestellt werden soll, und hierzu aus raumsparenden Gründen an Stelle des allgemein üblichen Zwei-Greifersystems ein Ein-Greifersystem zur Anwendung kommt, muß dessen Greifer 12 -wie noch später erläutert werden wird- eine dreidimensionale Bewegung ausführen, die sich aus einer vertikalen Bewegung, einer Schwingbewegung und einer im wesentlichen horizontal gerichteten oszillierenden Bewegung zusammensetzt.

Zum Antrieb des Greifers 12 ist ein auf der Hauptwelle 9 angeordnetes Kegelrad 26 vorgesehen, das mit einem Gegenrad 27 in.,Eingriff steht, das von einer im Gehäusemittelteil 4 gelagerten Zwischenwelle 28 aufgenommen ist. Diese trägt an ihrem unteren Ende ein Stirnrad 29, das mit einem Gegenrad 31 kämmt. Letzteres ist auf einer ebenfalls im Gehäusemittelteil 4 gelagerten Welle 32 angeordnet, deren unteres Ende ein Zahnriemenrad 33 trägt, das über einen Zahnriemen 34 mit einem Gegenrad 35 in Eingriff steht. Dieses ist auf einer zur Welle 32 parallel gerichteten und ebenfalls im Gehäusemittelteil 4 gelagerten Welle 36 angeordnet. Die beiden Wellen 32 und 36 laufen somit gleichsinnig um.

Auf der Welle 32 ist oberhalb des Zahnriemenrades 33 eine Zylinderkurve 37 und auf der Welle 36 eine Zylinderkurve 38 angeordnet. Während die Zylinderkurve 37 in noch später zu beschreibender Weise zur Erzielung einer oszillierenden Bewegung des Greifers 12 dient, dient die Zylinderkurve 38 zur Erzielung der Hubbewegung des Greifers 12 und damit zu einer Hubbewegung einer diesen tragenden und im wesentlichen parallel zu den Wellen 32, 36 angeordneten Greiferwelle 39. Diese ist zweiteilig ausgebildet und besteht aus einem Oberteil 41 und einem den Greifer 12 tragenden Unterteil 42. Zur Verbindung des im Gehäusemittelteil 4 drehund längsverschiebbar aufgenommenen Oberteils 41 mit dem Unterteil 42 dient ein Gelenkstück 43, das zwar innerhalb einer vertikalen Ebene eine Relativbewegung zwischen dem Oberteil 41 und dem Unterteil 42 zuläßt, dabei aber das Oberteil 41 drehfest mit dem Unterteil 42 verbindet.

Auf dem Oberteil 41 der Greiferwelle 39 ist ein Träger 44 für eine in eine Kurvennut 45 der Zylinderkurve 38 ragende Abtastrolle 46 angeordnet, deren Vertikalbewegungen über den Träger 44 auf das Oberteil 41 übertragen werden. Zur Übertragung der axialen Bewegung des Trägers 44 auf die Greiferwelle 39 ist dieser zwischen einem auf das Oberteil 41 aufgeklemmten Stellring 48 und einer im Abstand hierzu auf dem Oberteil 41 befestigten Anlaufscheibe 49 angeordnet.

Die Greiferwelle 39 ist im Träger 44 frei drehbar. Der Träger 44 weist einen prismatischen Ansatz 47 auf, der in einer (nicht näher dargestellten) Nut des Gehäusemittelteils 4 gleitet und den Träger 44 gegen Verdrehen sichert.
Die Zylinderkurve 38 bildet zusammen mit den zwischen ihr und dem Oberteil 41 der Greiferwelle 39 angeordneten Teilen einen Hubantrieb 50 für diese.

Zur Erzielung einer Schwingbewegung des Greifers 12 ist auf der Welle 36 oberhalb der Zylinderkurve 38 eine weitere Kurvenscheibe 52 angeordnet, die an ihrer unteren Stirnseite eine Nut 53 aufweist, in die eine Abtastrolle 54 eingreift. Die Abtastrolle 54 ist zwischen den Enden eines Hebels 55 angeordnet, dessen eines Ende um einen zur Welle 36 parallel gerichteten Zapfen 56 schwenkbar ist, der seinerseits in einer Buchse 57 gelagert ist, die im Gehäusemittelteil 4 befestigt ist. An das noch freie andere Ende des Hebels 55 ist eine Koppel 58 angelenkt, die mit einer Schwinge 59 gelenkig verbunden ist. Die Schwinge 59 ist von einer am oberen Ende des Oberteils 41 der Greiferwelle 39 angeordneten Buchse 61 gebildet, die zum Angriff der Koppel 58 einen hebelarmähnlichen Ansatz 62 aufweist. Die Schwinge 59 ist mit dem Oberteil 41 der Greiferwelle 39 drehfest verbunden, die hierzu im Bereich ihres oberen Endes ein Keilwellenprofil 63 aufweist, das in ein in der Buchse 61 vorgesehenes (nicht näher bezeichnetes) Gegenprofil eingreift. Durch das mit dem Gegenprofil zusammenwirkende Keilwellenprofil 63 wird einerseits die drehfeste Verbindung zwischen der Schwinge 59 und dem Oberteil 41 der Greiferwelle 39 gewährleistet, andererseits gleitet das Keilwellenprofil 63 während den vom Hubantrieb 50 eingeleiteten Hubbewegungen der Greiferwelle 39 in der in axialer Richtung festgelegten Schwinge 59. Die über die vorgenannte Getriebeanordnung von der Kurvenscheibe 52 abgeleiteten Bewegungen des Hebels 55 bewirken somit eine Schwingbewegung der Greiferwelle 39 um ihre eigene Achse, ohne hierbei ihre axiale Bewegbarkeit zu beeinträchtigen. Die Getriebeverbindung von der Kurvenscheibe 52 bis zur Greiferwelle 39 bzw zum Greifer 12 bildet einen Schwingantrieb 60 für den Greifer 12 bzw für die Greiferwelle 39.

Zur Erzielung der oszillierenden Bewegung des Unterteils 42 der Greiferwelle 39 weist die vorerwähnte Zylinderkurve 37 eine Kurvennut 64 für eine Abtastrolle 65 auf, deren vertikale Bewegungen über einen die Abtastrolle 65 aufnehmenden Träger 66 auf eine -zu noch später zu erläuterndem Zweck als Rohr ausgebildete- Schubstange 67 übertragen werden. Der Träger 66 weist einen prismatischen Ansatz 68 auf, der in einer (nicht näher dargestellten) Nut des Gehäusemittelteils 4 gleitet und den Träger 66 gegen Verdrehen sichert.

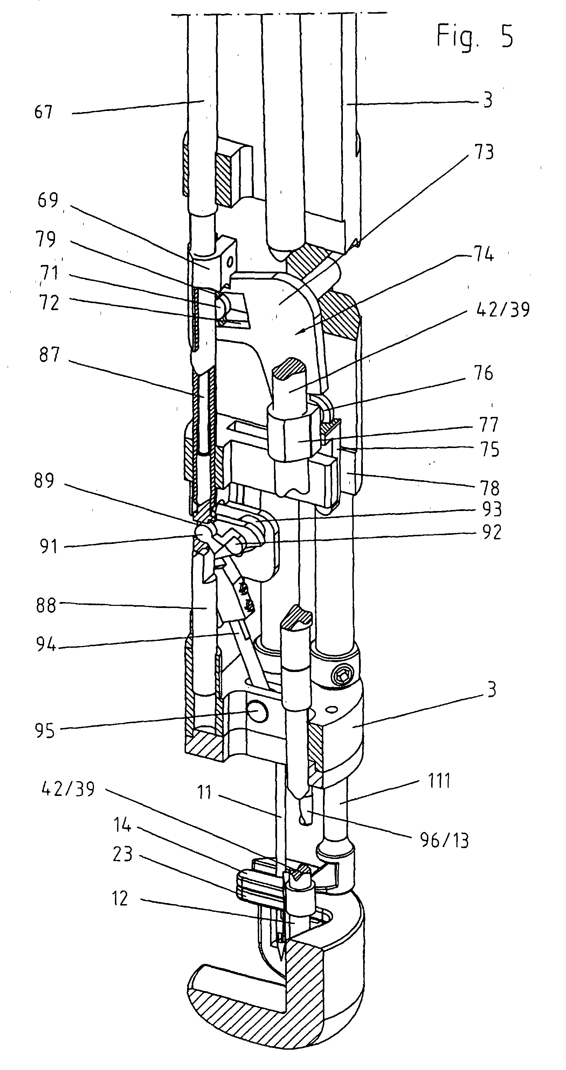

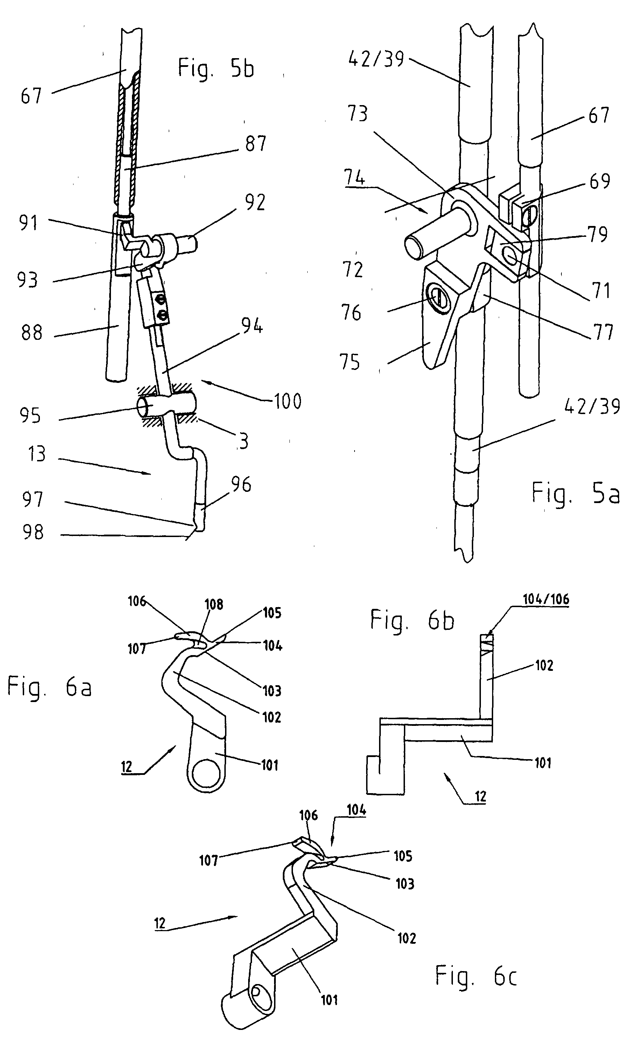

Die Schubstange 67 ist im Gehäuseschaft 3 geführt und weist im Bereich ihres unteren Endes (Fig. 5) einen von einem Klemmstück 69 getragenen und quer zur Schubstange 67 gerichteten Zapfen 71 auf, der einen Gleitstein 79 trägt. Dieser gleitet innerhalb einer Führungsnut 72, die an einem Arm 73 eines im Gehäuseschaft 3 gelagerten Winkelhebels 74 ausgebildet ist. Der andere Arm 75 des Winkelhebels 74 trägt ein Gelenkstück 76, das mit einer auf dem Unterteil 42 der Greiferwelle 39 längsverschiebbar angeordneten Hülse 77 verbunden ist.
Hierdurch wird von der Hubbewegung der Schubstange 67 eine oszillierende Bewegung des Unterteils 42 der Greiferwelle 39 um das zwischen diesem und dem Oberteil 41 der Greiferwelle 39 angeordnete Gelenkstück 43 abgeleitet. Aufgrund des relativ großen Schwenkradius für das Unterteil 42 ist der Hubanteil dieser oszillierenden Bewegung funktionell vernachlässigbar, sodaß die von der Schubstange 67 abgeleitete oszillierende Bewegung des Unterteils 42 als Horizontalbewegung angesehen werden kann.
Zur sicheren Führung des Unterteils 42 der Greiferwelle 39 kann der Arm 75 des Winkelhebels 74 über das Gelenkstück 76 hinaus verlängert sein, und innerhalb einer im Gehäuseschaft 3 vorgesehene Führungsnut 78 gleiten. Hierdurch wird das Unterteil 42 der Greiferwelle 39 gegen quer zu ihrer Horizontalbewegung gerichtete Kräfte gesichert.
Die Getriebeverbindung von der Zylinderkurve 37 zum Unterteil 42 der Greiferwelle 39 wird als Horizontalantrieb 80 der Greiferwelle 39 bzw des Greifers 12 bezeichnet.

Oberhalb der Zylinderkurve 38 (Fig. 2) ist auf der diese aufnehmenden Welle 36 eine weitere Kurvenscheibe 81 angeordnet, die eine Kurvennut 82 für eine von einem Träger 83 aufgenommene Abtastrolle 84 aufweist. Der Träger 83 ist auf einem im Gehäusemittelteil 4 befestigten, im wesentlichen vertikal gerichteten Führungszapfen 85 längsverschiebbar gelagert und weist einen prismatischen Ansatz 86 auf, der in eine im Gehäusemittelteil 4 vorgesehene (nicht näher dargestellte) Führungsnut ragt und den Träger 83 gegen Verdrehen sichert.

Am Träger 83 ist eine Stange 87 befestigt, die durch die rohrförmig ausgebildete Schubstange 67 hindurch bis zu deren unterem Ende geführt ist. Im Bereich ihres unteren Endes ist die Stange 87 mit einem Zapfen 88 verbunden (Fig. 5), der in einem im Gehäuseschaft 3 vorgesehenen (nicht dargestellten) Lager axial geführt ist. Im Zapfen 88 ist ein im wesentlichen rechteckförmiger Durchbruch 89 vorgesehen, in den das freie Ende eines scheibenförmig ausgebildeten Hebels 91 ragt, der auf einer im Gehäuseschaft 3 gelagerten und im wesentlichen horizontal gerichteten Welle 92 angeordnet ist. Hierdurch wird die von der Kurvenscheibe 81 eingeleitete Hubbewegung des Stiftes 87 in eine Schwenkbewegung des Hebels 91 und somit der Welle 92 gewandelt.

Auf der Welle 92 (Fig. 5b) ist eine Kurbel 93 angeordnet, an die ein Ende einer Gleitstange 94 angelenkt ist, die in einem im Gehäuseschaft 3 gelagerten Dreh-Schiebe-Gelenk 95 verschieblich angeordnet ist. Am noch freien Ende der Gleitstange 94 ist ein Schaft 96 des eigentlichen Fadenlenkers 13 für den oberen Schenkel der Fadenschlinge ausgebildet, der mit einer Aufnahmenut 97 versehen ist, die im wesentlichen V-förmig ausgebildet ist und eine schräg gerichtete Auflagefläche 98 für die Fadenschlinge aufweist. Der Schaft 96 ist im wesentlichen parallel zur Greiferwelle 39 gerichtet, wobei die Schräglage seiner Aufnahmenut 97 so gewählt ist, daß sie zum Zeitpunkt der Aufnahme der Fadenschlinge im wesentlichen parallel zu dieser verläuft. Aufgrund der vorbeschriebenen getriebemäßigen Anordnung führt der Fadenlenker 13 eine aus einer vertikalen und einer horizontalen Komponente zusammengesetzte Bewegung aus, wobei die horizontale Komponente im wesentlichen parallel zur Richtung der zu bildenden Naht erfolgt.
Die Kurvenscheibe 81 bildet zusammen mit den zwischen ihr und dem Schaft 96 angeordneten Teilen einen Lenkertrieb 100 für den Fadenlenker 13.

Der am unteren Ende der Greiferwelle 39 angeordnete Greifer 12 (Fig. 6a, 6b und 6c) ist mit dieser fest verbunden und nimmt somit an allen Bewegungskomponenten (vertikale Bewegung, Schwingbewegung und horizontal gerichtete oszillierende Bewegung) der Greiferwelle 39 teil.

Der Greifer 12 weist einen zur Achse seiner Aufnahmebohrung im wesentlichen parallel gerichteten Bereich 101 auf, an den sich ein Schaft 102 anschließt. Der Schaft 102 geht in einen Steg 103 über, an dessen freiem Ende eine in üblicher Weise die unterhalb des Nähgutes gebildete Fadenschlinge aufnehmende Hauptklinge 104 mit einer Spitze 105 ausgebildet ist. Der Hauptklinge 104 gegenüberliegend ist am Schaft 102 eine Gegenklinge 106 ausgebildet, deren Spitze 107 zur Spitze 105 der Hauptklinge 104 im wesentlichen entgegengesetzt gerichtet ist. Die Gegenklinge 106 dient in noch zu beschreibender Weise, zum Verhindern eines Abgleitens der von der Hauptklinge 104 erfaßten Fadenschlinge und weist im Bereich Ihres Überganges zum Schaft 102 eine sich über ihre gesamte Breite erstreckende Aufnahmenut 108 für den unteren Schenkel der Fadenschlinge auf.

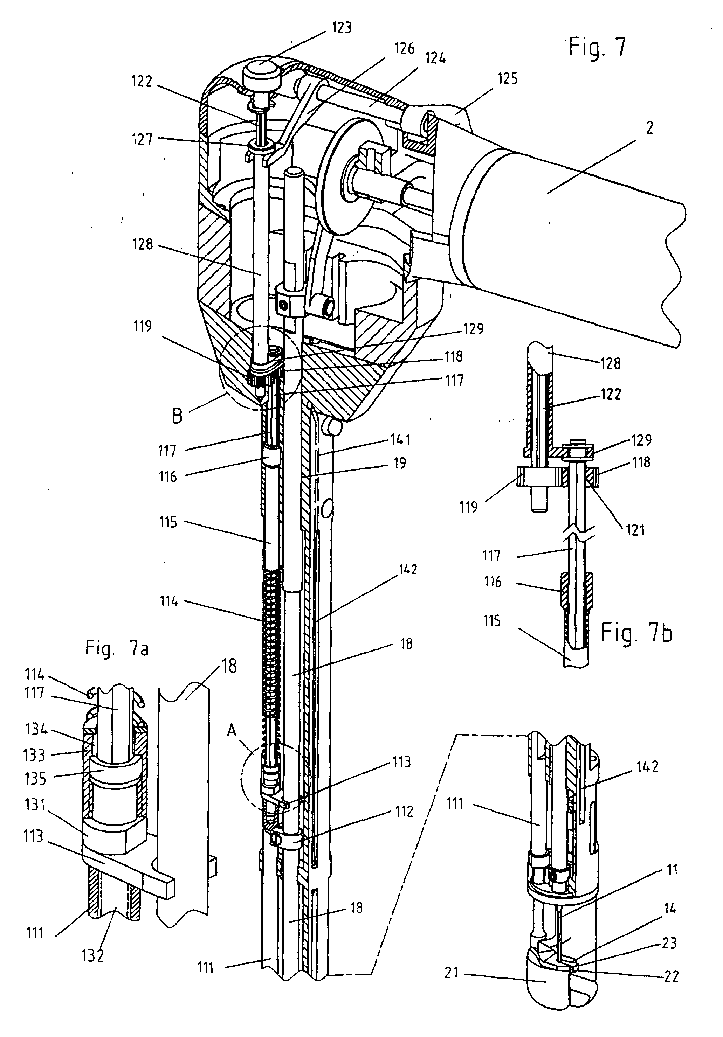

Der zum Festhalten des Nähgutes auf der Stichplatte 21 dienende Niederhalter 14 (Fig. 7, 7a und 7b) ist am unteren Ende einer im Gehäuseschaft 3 verschieblich angeordneten Druckstange 111 angeordnet, die im wesentlichen parallel zur Nadelstange 18 gerichtet ist. Um zumindest während eines Teils der ausgestochenen Phase der Nadel 11 den Niederhalter 14 zur Erleichterung der Vorschubbewegug des Nähgutes um einen bestimmten Betrag periodisch anheben zu können, ist auf der Nadelstange 18 ein Mitnehmer 112 angeordnet, der einen im oberen Bereich der Druckstange 111 befestigten Anschlag 113 untergreift und diesen zumindest während eines Teils der Hubbewegung der Nadelstange 18 mitnimmt. Die Position des Mitnehmers 112 auf der Nadelstange 18 kann dabei so gewählt werden, daß der Niederhalter 14 symmetrisch zum oberen Umkehrpunkt der Nadel angehoben und abgesenkt wird.

Der Anschlag 113 ist im Bereich seines von der Druckstange 111 abgekehrten Endes gabelförmig ausgebildet und umgreift mit diesem die Nadelstange 18, wodurch die Druckstange 111 gleichzeitig auch gegen Drehbewegungen gesichert ist. Der die Druckstange 111 umgebende Bereich des Anschlages 113 dient gleichzeitig zur Abstützung des unteren Endes einer Druckfeder 114, deren oberes Ende sich gegen eine Buchse 115 abstützt, die ihrerseits an der Unterseite einer Gewindebuchse 116 anliegt. Diese arbeitet mit einem im Gehäuseschaft 3 vorgesehenen Innengewinde zusammen. Zur axialen Verschiebung der Gewindebuchse 116 und somit zur Veränderung der Kraft der Druckfeder 114 ist die Gewindebuchse 116 mit einem nicht näher dargestellten Innensechskant versehen und von einer Stange 117 durchdrungen, die einen dem Innensechskant entsprechenden Querschnitt aufweist. Die Stange 117 ist innerhalb der Gewindebuchse 116 zwar längsverschieblich angeordnet, mit dieser aber drehfest verbunden. Oberhalb der Gewindebuchse 116 ist im Gehäuseschaft 3 ein in einer im wesentlichen horizontalen Ebene umlaufendes Stirnrad 118 vorgesehen, das mit einem Gegenrad 119 kämmt. Das Stirnrad 118 ist ebenfalls von der Stange 117 durchdrungen, jedoch kann deren Querschnitt, da die Stange 117 nur durch das Stirnrad 118 frei hindurchgeführt ist, kleiner sein als die hierfür im Stirnrad 118 vorgesehene axiale Bohrung 121. Das Gegenrad 119 ist mit einer Achse 122 verbunden, die im Gehäusemittelteil 4 gelagert und im wesentlichen parallel zur Druckstange 111 gerichtet ist. Die im Gehäusemittelteil 4 axial festgelegte Achse 122 trägt an ihrem oberen Ende ein Einstellhandhabe 123, die außerhalb des Gehäusemittelteils 4 angeordnet ist. Durch Drehen der Einstellhandhabe 123 wird die Gewindebuchse 116 und damit die Buchse 115 innerhalb des Gehäuseschaftes 3 axial verschoben und durch die damit verbundene Veränderung der Federlänge die Kraft der Druckfeder 114 verändert.

Zum manuellen Heben und Absenken des Niederhalters 14 ist im Gehäusemittelteil 4 eine im wesentlichen horizontal gerichtete Achse 124 gelagert, mit der sowohl eine aus dem Gehäusemittelteil 4 herausragende weitere Handhabe 125, als auch ein Schwenkhebel 126 fest verbunden sind. Der an seinem freien Ende abgekröpfte und gabelförmig ausgebildete Schwenkhebel 126 untergreift einen Bund 127 einer die Achse 122 umgebenden, mit dieser aber nicht verbundenen Büchse 128, die an ihrem unteren Ende einen mit ihr fest verbundenen Bügel 129 trägt. Die Achse 122 ist zwar durch den Bügel 129 hindurchgeführt, jedoch ist diese unabhängig vom Bügel 129 frei drehbar. Der Bügel 129 ist an seinem noch freien Ende mit dem Ende der aus dem Stirnrad 118 herausragenden Stange 117 verbunden. Diese Verbindung ist dergestalt, daß die Stange 117 gegenüber dem Bügel 129 frei drehbar ist, beide in axialer Richtung aber fest miteinander verbunden sind.

Das aus der Buchse 115 herausragende Ende der Stange 117 ist durch die Druckfeder 114 hindurch bis zum Anschlag 113 geführt und bewirkt eine Zentrierung derselben. In das obere Ende der Druckstange 111 ist ein mit einem Bund 131 versehener Gewindezapfen 132 eingeschraubt, dessen oberes Ende seinerseits in eine Gewindehülse 133 einschraubbar ist. Die Gewindehülse 133 ist mit einer axial gerichteten Bohrung 134 versehen, durch die das untere Ende der Stange 117 hindurchgeführt und mittels eines an der Stange 117 ausgebildeten Bundes 135 in der Gewindehülse 133 axial festgelegt ist.
Da das gabelförmig ausgebildete Ende des Anschlages 113 den Bund 131 des Gewindezapfens 132 untergreift, kann der Niederhalter 14 durch Verschwenken der Handhabe 125 manuell angehoben und abgesenkt werden.

Zur Zuführung eines Fadens von einer außerhalb des Gehäuses gelegenen Fadenvorratsstelle zur Nadel 11 ist am Gehäuseschaft 3 eine Bohrung 141 (Fig. 2) vorgesehen, die in ein innerhalb des Gehäuseschaftes 3 angeordnetes Fadenführungsrohr 142 mündet. Der Faden tritt am unteren Ende dieses Fadenführungsrohres 142 aus diesem aus und wird von da zur Nadel 11 geführt.
Der Gehäuseschaft 3 sowie sämtliche an und in diesem angeordnete Funktionsteile sind von einem auf den Gehäuseschaft 3 aufschiebbaren Schutzrohr 143 umgeben, das im oberen Bereich des Gehäuseschaftes 3 fixierbar ist.
Da beim Einführen des Gehäuseschaftes 3 in den Körper sowohl die Stichbildewerkzeuge 11, 12, als auch die Stichplatte 21 und der Niederhalter 14, als auch der Fadenlenker 13, nicht gegenüber dem Schutzrohr 143 vorstehen sollen, vielmehr in dieses zurück gezogen sein sollen, ist das Schutzrohr 143 am Gehäuseschaft 3 in einer ersten Position arretierbar, in der dieses gegenüber den vorerwähnten Funktionsteilen vorsteht.
Zur Bildung der Naht ist das Schutzrohr 143 in einer zweiten Position arretierbar, in der die vorerwähnten Funktionsteile ihrerseits gegenüber dem Schutzrohr 143 vorstehen. Dabei können beide Arretierungen des Schutzrohres 143 im oberen Bereich des Gehäuseschaftes 3 angeordnet sein und in üblicher Weise von federbelasteten Kugeln gebildet sein.

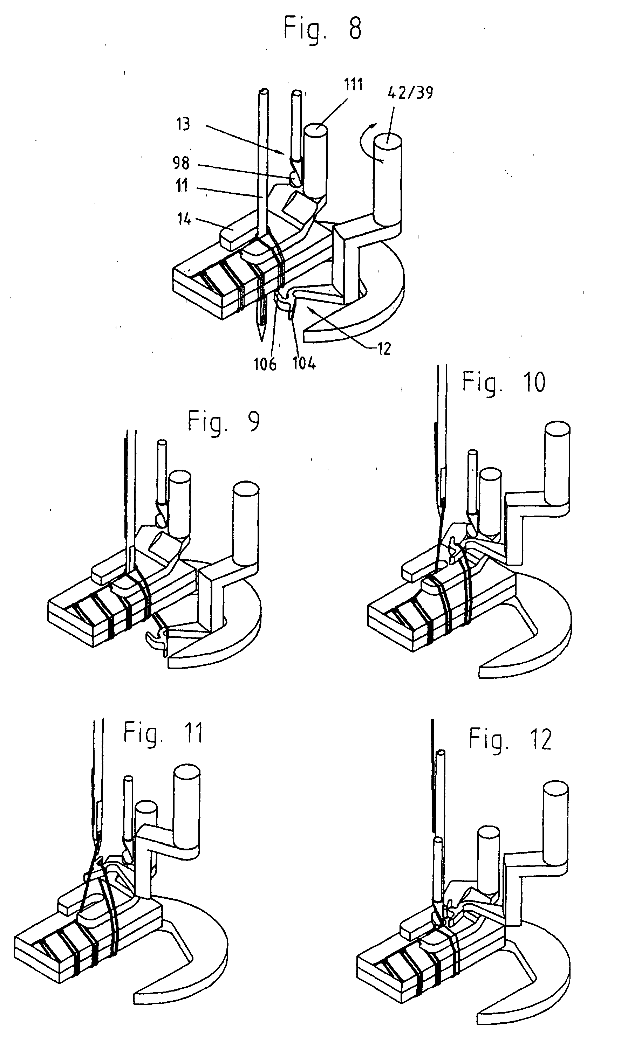

Für die Erläuterung der Stichbildung wird nachstehend von der unteren Totpunktlage der Nadel 11 ausgegangen die in Fig. 8 dargestellt ist.

Die vom Nadelantrieb 20 in bekannter Weise angetriebene Nadel 11 hat dabei die vom Greifer 12 nach oben geführte Fadenschlinge durchstochen und hält diese oberhalb des Niederhalters 14. Die Greiferwelle 39 und der damit verbundene Greifer 12 befinden sich dabei noch auf ihrer nach abwärts gerichteten Bewegung, wobei gleichzeitig die Hauptklinge 104 des Greifers 12 und damit die Spitze 105 der Hauptklinge 104 -bezogen auf Fig. 8- durch den Schwingantrieb 60 in Richtung des in Fig. 8 dargestellten Pfeiles zur Nadel 11 hin geschwenkt wird. Diese Schwenkrichtung wird nachfolgend als "im Uhrzeigersinn" gerichtete Schwenkbewegung des Greifers 12 bzw der Greiferwelle bezeichnet. Dabei ist das Gelenkstück 43 derart ausgelenkt, daß sich das Unterteil 42 der Greiferwelle 39 im Bereich des von der Nadel 11 abgekehrten Endes seiner horizontal gerichteten Auslenkbewegung befindet. Der Fadenlenker 13 befindet sich dabei in seiner oberen Stellung, die seiner Ruhestellung entspricht.

Während der Ausführung des Schlingenhubes der Nadel 11 wird die Spitze 105 der Hauptklinge 104 in Fortsetzung ihrer im Uhrzeigersinn erfolgenden Schwenkbewegung durch den Schwingantrieb 60 in die unterhalb der Stichplatte 21 gebildete Fadenschlinge bewegt, sodaß diese von der Hauptklinge 104 aufgenommen wird und auf dieser hängt. Der Fadenlenker 13 befindet sich weiterhin in seiner Ruhestellung. Der Horizontalantrieb 80 hat das Unterteil 42 der Greiferwelle 39 in Richtung zur Nadel 11 zu schieben begonnen, wodurch die Fadenschlinge auf der Hauptklinge 104 in Richtung zum Schaft 102 des Greifers 12 bewegt wird.

Im weiteren Verlauf der Stichbildung wird der Greifer 12 mit der von der Hauptklinge 104 aufgenommenen Fadenschlinge durch den Schwingantrieb 60 entgegen dem Uhrzeigersinn geschwenkt und gelangt in eine seitlich des Nähgutes gelegene Stellung (Fig.9). Anschließend bewegt der Hubantrieb 50 den Greifer 12 von seiner höhenmäßig unterhalb des Nähgutes gelegenen Position in eine oberhalb des Nähgutes gelegene Ebene, in der die Hauptklinge 104 sich höhenmäßig in einer Art Zwischenstellung befindet.
Von hier aus wird die Hubbewegung des Greifers 12 unter gleichzeitigem Einsetzen des Schwingantriebes 60 fortgesetzt, sodaß die Hauptklinge 104 noch weiter angehoben und dabei gleichzeitig hinter die Achse der zwischenzeitlich nach oben bewegten Nadel 11 geführt wird. Die Hauptklinge 104 kreuzt dabei die Ebene der Bewegungsbahn der Nadel 11, die sich im Bereich ihres oberen Totpunktes befindet (Fig. 10).
Während dieser im Uhrzeigersinn erfolgenden Schwenkbewegung des Greifers 12 erfolgt bei durch die Nadelstange 18 angehobenem Niederhalter 14 eine manuelle Vorschubbewegung des Nähgutes.

Die noch immer von der Hauptklinge 104 getragene Fadenschlinge liegt am Rand des Nähgutes an und verläuft von da zur Spitze 105 der Hauptklinge 104. Die Fadenschlinge hat dabei den Trend, von der Hauptklinge 104 abzugleiten. Dies wird durch die Gegenklinge 106 verhindert, wobei einer der Schenkel der Fadenschlinge in die Aufnahmenut 108 der Gegenklinge 106 gleitet und in dieser fixiert wird. Der Fadenlenker 13 ist auch weiterhin noch in seiner Ruhestellung. Der Horizontalantrieb 80 lenkt das Unterteil 42 der Greiferwelle 39 in Richtung zur Nadel 11 hin aus, wobei die auf der Hauptklinge 104 hängende Fadenschlinge zur Achse der Nadel 11 hin bewegt wird. Die sich auf ihrer nach abwärts gerichteten Bewegung befindliche Nadel 11 sticht in das durch die Schräglage der Aufnahmenut 108 des Greifers 12 gespreizte Fadendreieck ein (Fig. 11).

Nach erfolgtem Abstechen der Fadenschlinge durch die Nadel 11 bewegt der Horizontalantrieb 80 das Unterteil 42 der Greiferwelle 39 entgegen dessen vorheriger Bewegung von der Nadel 11 in Richtung seines hinteren Umkehrpunktes weg. Hierdurch wird die Bewegungsbahn für eine dem Uhrzeigersinn entgegengesetzte Schwenkbewegung der Hauptklinge 104 zur Vermeidung einer Kollision zwischen dieser und der Nadel 11 frei, sodaß die Hauptklinge 104 durch den Schwingantrieb 60 von ihrer -bezogen auf den Rand des Nähgutes- hinter der Achse der Nadel 11 gelegenen Position in eine seitlich des Randes des Nähgutes gelegene Position bewegt werden kann.

Vor Beginn dieser Schwenkbewegung der Hauptklinge 104 setzt die durch den Lenkertrieb 100 bewirkte, schräg nach abwärts gerichtete, Bewegung des Fadenlenkers 13 ein, wodurch der obere Schenkel der Fadenschlinge vom Fadenlenker 13 erfaßt und durch dessen V-förmige Aufnahmenut 97 fixiert wird. Der erfaßte Schenkel der Fadenschlinge liegt dabei auf der Schrägfäche 98 des Fadenlenkers 13 auf. Dieser führt anschließend seine die Fadenschlinge aus dem Bereich der Hauptklinge 104 führende Arbeitsbewegung aus und verlagert den oberen Schenkel der Fadenschlinge in Nährichtung. Hierdurch kann die Hauptklinge 104 durch den Schwingantrieb 60 entgegen dem Uhrzeigersinn geschwenkt werden, ohne hierbei mit der vom Fadenlenker 13 getragenen Fadenschlinge zu kollidieren.

Sobald die Hauptklinge 104 sich über die noch vom Fadenlenker 13 aufgenommene Fadenschlinge hinweg bewegt hat (Fig.12), wird der Fadenlenker 13 durch den Lenkertrieb 100 in seine Ruhestellung zurück bewegt. Da diese aus einer vertikalen und einer horizontalen Bewegungskomponente gebildet ist und die horizontale Komponente in Richtung der Spitze der Aufnahmenut 97 gerichtet ist, gleitet die Fadenschlinge zu Beginn der Rückwärtsbewegung des Fadenlenkers 13 von der Auflagefläche 98 der Aufnahmenut 97 ab; der Fadenlenker 13 kehrt jetzt in seine Ruhestellung zurück. Gleichzeitig verlagert der Horizontalantrieb 80 das Unterteil 42 der Greiferwelle 39 zum von der Nadel 11 abgekehrten Umkehrbereich seiner Auslenkbewegung. Sobald der Greifer 12 seine entgegen dem Uhrzeigersinn gerichtete Schwenkbewegung vollendet hat, setzt der Hubantrieb ein und führt den Greifer 12 in seine unterhalb des Nähgutes gelegene Schwingebene zurück. Der Stichbildevorgang kann sich jetzt wiederholen.

## Patentansprüche

1. Endoskopische Nähmaschine mit einem Gehäuse, bestehend aus einem Gehäuseoberteil (2,4) zur Aufnahme von Antrieben (20, 50, 60,und 80) für die Stichbildewerkzeuge (11,12), einem sich an dieses anschließenden Gehäuseschaft (3) zur Aufnahme von Mitteln zur Übertragung der von den Antrieben (20, 50, 60,und 80) erzeugten Bewegungen auf die Stichbildewerkzeuge (11,12) die mindestens eine dem Gehäuseschaft koaxiale, fadenführende Nadel (11) und einen mit dieser zusammenwirkenden Greifer (12) aufweisen, **dadurch gekennzeichnet, dass** der Greifer nach dem Erfassen der durch die Nadel (11) gebildeten Fadenschlinge entlang einer mehrdimensionalen Bewegungsbahn von seiner unterhalb des Nähgutes gelegenen, die Fadenschlinge erfassenden Position in eine oberhalb des Nähgutes gelegene Position bewegbar ist, in der das von der auf die Nähgutoberseite geführten Fadenschlinge gebildete Fadendreieck die Projektion der Nadelbahn umschließt.

2. Endoskopische Nähmaschine nach Anspruch 1, **dadurch gekennzeichnet, daß**
die Bewegungsbahn des Greifers (12) aus einer unterhalb des Nähgutes erfolgenden ersten Schwingbewegung, einer sich hieran anschließenden Hubbewegung und einer zweiten Schwingbewegung zusammengesetzt ist.

3. Endoskopische Nähmaschine nach Anspruch 1, **dadurch gekennzeichnet, daß**
daß die zum Erfassen der Fadenschlinge dienende erste Schwingbewegung des Greifers (12) um eine Achse (41) erfolgt, deren Abstand zur Nadel (11) größer ist, als der Abstand der Nadel (11) zur Achse (42) um die die zur Positionierung der Fadenschlinge auf dem Nähgut dienende zweite Schwingbewegung erfolgt.

4. Endoskopische Nähmaschine nach Anspruch 1, **dadurch gekennzeichnet, daß**
die den Greifer (12) tragende Greiferwelle (39) im Gehäuse (3,4) längsverschiebbar und drehbar angeordnet ist und ihr ein Schwingantrieb (60) und ein Hubantrieb (50) zugeordnet ist, deren Antriebsmittel (Kurvenscheiben 38,52) auf ein und derselben Welle (38) angeordnet sind, die parallel zur Greiferwelle (39) gerichtet ist.

5. Endoskopische Nähmaschine nach Anspruch 4, **dadurch gekennzeichnet, daß**
das Abtriebsteil (Träger 44) des Hubantriebes (50) in axialer Richtung mit der Greiferwelle (39) starr verbunden ist und diese im Abtriebsteil (Träger 44) frei drehbar ist.

6. Endoskopische Nähmaschine nach Anspruch 4, **dadurch gekennzeichnet, daß**
das Abtriebsteil (Buchse 59) des Schwingantriebes (60) mit der Greiferwelle (39) drehfest verbunden ist und diese in axialer Richtung im Abtriebsteil (Buchse 59) längsverschieblich ist.

7. Endoskopische Nähmaschine nach Anspruch 1, **dadurch gekennzeichnet, daß**
die Greiferwelle (39) von einem Oberteil (41) und einem Unterteil (42) gebildet ist und beide mittels eines Gelenkstückes (43) derart miteinander verbunden sind, daß das Unterteil (42) in horizontaler Richtung relativ zum Oberteil (41) bewegbar ist.

8. Endoskopische Nähmaschine nach Anspruch 7, **dadurch gekennzeichnet, daß**
zur Erzielung einer Relativbewegung zwischen dem Oberteil (41) und dem Unterteil (42) ein Horizontalantrieb (80) vorgesehen ist, der eine von einer Kurvenscheibe (37) abgeleitete Hubbewegung einer Schubstange (67) bewirkt, die über eine Antriebsverbindung (69 bis 76) als Horizontalbewegung auf das Unterteil (42) übertragbar ist.

9. Endoskopische Nähmaschine nach Anspruch 7 und 8, **dadurch gekennzeichnet, daß**
die Kurvenscheibe (37) des Horizontalantriebes (80) auf einer zur Welle (38) parallel gerichteten Welle (32) angeordnet ist, und oberhalb der Kurvenscheiben für den Schwing- und Hubantrieb eine weitere Kurvenscheibe (81) zum Antrieb eines Fadenlenkers (13) trägt.

10. Endoskopische Nähmaschine nach Anspruch 9, **dadurch gekennzeichnet, daß**
die Kurvenscheibe (81) eine Stange (87) betätigt, die innerhalb der Schubstange (67) längsverschieblich angeordnet ist, wobei deren aus der Schubstange (67) hervorstehendes Ende dem Fadenlenker (13) über eine Antriebsverbindung (88 bis 95) eine eine vertikale und eine horizontale Bewegungskomponente aufweisende Bewegung erteilt.

11. Endoskopische Nähmaschine nach einem oder mehreren der vorangehenden Ansprüche 1, **dadurch gekennzeichnet, daß**
der Fadenlenker (13) eine im wesentlichen V-förmige, schräg nach aufwärts gerichtete Aufnahmenut (97) für einen Schenkel des Fadenschlinge aufweist, wobei die Schräglage der Aufnahmenut (97) zum Zeitpunkt der Aufnahme der Fadenschlinge im wesentlichen parallel zu dieser verläuft.

12. Endoskopische Nähmaschine nach Anspruch 1, **dadurch gekennzeichnet, daß**
der Greifer (12) eine Gegenklinge (106) aufweist, deren Spitze (107) zur Spitze (105) der Hauptklinge (104) im wesentlichen entgegengesetzt gerichtet ist.

## Claims

1. Endoscopic suturing machine having a housing, comprising an upper housing part (2, 4) for accommodating drives (20, 50, 60 and 80) for the stitch-forming tools (11, 12), a housing shaft (3) communicating with said upper housing part for accommodating means for the transmission of the movements, produced by the drives (20, 50, 60 and 80), to the stitch-forming tools (11, 12), which have at least one thread-guiding needle (11), which is coaxial with the housing shaft, and one gripper (12) which co-operates with said needle, **characterised in that**, after gripping of the thread loop formed by the needle (11), the gripper is displaceable along a multi-dimensional displacement path from its thread loop gripping position, which is situated beneath the material being sutured, into a position which is situated above the material being sutured, and in which position the thread triangle, formed by the thread loop guided on the upper side of the material being sutured, encloses the projection of the needle path.

2. Endoscopic suturing machine according to claim 1, **characterised in that** the displacement path of the gripper (12) is composed of a first pivotal movement which is effected beneath the material being sutured, a lifting movement which communicates with said first pivotal movement, and a second pivotal movement.

3. Endoscopic suturing machine according to claim 1, **characterised in that** the first pivotal movement of the gripper (12), serving to detect the thread loop, is effected about a spindle (41), the spacing between said spindle and the needle (11) being greater than the spacing between the needle (11) and the spindle (42), about which the second pivotal movement, serving to position the thread loop on the material being sutured, is effected.

4. Endoscopic suturing machine according to claim 1, **characterised in that** the gripper shaft (39), carrying the gripper (12), is disposed in the housing (3, 4) in a longitudinally displaceable and rotatable manner, and a pivotal drive (60) and a lifting drive (50) are associated with said shaft, the driving means (cam discs 38, 52) of which drives are disposed on one and the same shaft (36) which is orientated parallel to the gripper shaft (39).

5. Endoscopic suturing machine according to claim 4, **characterised in that** the driven part (support 44) of the lifting drive (50) is rigidly connected in the axial direction to the gripper shaft (39), and said gripper shaft is freely rotatable in the driven part (support 44).

6. Endoscopic suturing machine according to claim 4, **characterised in that** the driven part (bush 59) of the pivotal drive (60) is connected to the gripper shaft (39) in a non-rotatable manner, and said gripper shaft is longitudinally displaceable in the axial direction in the driven part (bush 59).

7. Endoscopic suturing machine according to claim 1, **characterised in that** the gripper shaft (39) is formed by an upper part (41) and a lower part (42), and the two parts are interconnected by means of a pivotable part (43) in such a manner that the lower part (42) is displaceable in the horizontal direction relative to the upper part (41).

8. Endoscopic suturing machine according to claim 7, **characterised in that** a horizontal drive (80) is provided in order to achieve a relative movement between the upper part (41) and the lower part (42), and causes a lifting movement of a push-rod (67), which movement is derived from a cam disc (37) and is transmittable via a drive connection (69 to 76) to the lower part (42) as a horizontal movement.

9. Endoscopic suturing machine according to claims 7 and 8, **characterised in that** the cam disc (37) of the horizontal drive (80) is disposed on a shaft (32) orientated parallel to the shaft (36), and said disc carries, above the cam discs for the pivotal and lifting drives, an additional cam disc (81) for driving a thread guide (13).

10. Endoscopic suturing machine according to claim 9, **characterised in that** the cam disc (81) actuates a rod (87), which is disposed in a longitudinally displaceable manner within the push-rod (67), the end of said rod, which protrudes from the push-rod (67), imparting to the thread guide (13), via a drive connection (88 to 95), a movement which has a vertical component and a horizontal component.

11. Endoscopic suturing machine according to one or more of the preceding claims, **characterised in that** the thread guide (13) has a substantially V-shaped receiving groove (97), which is orientated inclinedly upwardly, for accommodating a portion of the thread loop, the inclined position of the receiving groove (97) extending substantially parallel to the thread loop at the time when said thread loop is received.

12. Endoscopic suturing machine according to claim 1, **characterised in that** the gripper (12) has a counter-blade (106), the tip (107) of which is orientated substantially in opposition to the tip (105) of the principal blade (104).

## Revendications

1. Machine à suture endoscopique comprenant un boîtier constitué d'un boîtier supérieur (2, 4) destiné à loger les entraînements (20, 50, 60 et 80) pour les outils (11, 12) de formation des points, d' un boîtier (3) en forme de fût qui prolonge le précédent boîtier afin de loger des moyens de transmission des mouvements générés par les entraînements (20, 50, 60 et 80) sur les outils (11, 12) de formation des points, lesquels outils présentent au moins une aiguille (11) guidant le fil, coaxiale par rapport au boîtier en forme de fût et une griffe (12) agissant conjointement avec celle-ci, **caractérisée en ce que** la griffe puisse être déplacée, après saisie de la boucle de fil formée par l'aiguille (11), le long d'une trajectoire de déplacement sur plusieurs dimensions allant de sa position sous le tissu à suturer, saisissant la boucle de fil, jusqu'à une position au-dessus du tissu à suturer, dans laquelle le triangle de fil, formé par la boucle de fil guidée à la surface du tissu à suturer, englobe la projection de la trajectoire de l'aiguille.

2. Machine à suture endoscopique selon la revendication 1 **caractérisée en ce que** la trajectoire de déplacement de la griffe (12) se décompose en un premier mouvement d'oscillation se produisant sous le tissu suturer, lequel est suivi d'un mouvement de levée et d'un second mouvement d'oscillation.

3. Machine à suture endoscopique selon la revendication 1 **caractérisée en ce que** le premier mouvement d'oscillation de la griffe (12) servant à saisir la boucle de fil se fait autour d' un axe (41) dont l'intervalle par rapport à l'aiguille ( 11) est plus grand que l'intervalle de l'aiguille (11) par rapport à l'axe (42) autour duquel se fait le second mouvement d' oscillation servant à positionner la boucle de fil sur le tissu à suturer.

4. Machine à suture endoscopique selon la revendication 1 **caractérisée en ce que** l'arbre (39) supportant la griffe (12) est disposé dans le boîtier (3, 4) de manière à pouvoir être déplacé longitudinalement et en rotation et qu' il comporte un mécanisme (60) d'oscillation et un mécanisme (50) de levée, dont les moyens de commande (cames 38, 52) sont placés sur un seul et même arbre (38), parallèle à l'arbre (39) de la griffe.

5. Machine à suture endoscopique selon la revendication 4 **caractérisée en ce que** la pièce d'entraînement (support 44) du mécanisme de levée (50) est fermement reliée dans le sens axial avec l'arbre (39) de la griffe et **en ce que** celui-ci peut tourner librement dans la pièce d'entraînement (support 44).

6. Machine à suture endoscopique selon la revendication 4 **caractérisée en ce que** la pièce d'entraînement (douille 59) du mécanisme (60) d'oscillation est fermement' reliée à l'arbre (39) de la griffe de manière non rotative et **en ce que** celui-ci est décalable longitudinalement, dans le sens axial, dans la pièce d'entraînement (douille 59).

7. Machine à suture endoscopique selon la revendication 1 **caractérisée en ce que** l'arbre (39) de la griffe est formé d'une partie supérieure (41) et d'une partie inférieure (42) et **en ce que** les deux sont reliées au moyen d'une pièce d'articulation (43) de telle sorte que la partie inférieure (42) puisse être déplacée selon un mouvement relatif, horizontalement par rapport à la pièce supérieure (41).

8. Machine à suture endoscopique selon la revendication 7 **caractérisée en ce que** l'on prévoit, afin d'obtenir un mouvement relatif entre la partie supérieure (41) et la partie inférieure (42), un entraînement horizontal (80) qui provoque, par l'action d'une came (37), un mouvement de levée d'une bielle (67), lequel peut être transmis en tant que mouvement horizontal sur la pièce inférieure (42) au moyen d'un mécanisme de jonction (69 à 76).

9. Machine à suture endoscopique selon la revendication 7 et 8 **caractérisée en ce que** la came (37) du mécanisme horizontal (80) est disposée sur un arbre (32) orienté parallèlement à l'arbre (38) et supporte au-dessus des cames du mécanisme d'oscillation et de levée, une autre came (81) destinée à entraîner un guide-fil (13).

10. Machine à suture endoscopique selon la revendication 9 **caractérisée en ce que** la came (81) actionne une tige (87) qui est logée de manière à être décalable longitudinalement à l'intérieur de la bielle (67), l'extrémité dépassant de la bielle (67) transmettant au guide-fil (13), par le biais d'un mécanisme de jonction (88 à 95), un mouvement se décomposant en un mouvement vertical et un mouvement horizontal.

11. Machine à suture endoscopique selon une ou plusieurs des revendications 1 précédentes **caractérisée en ce que** le guide-fil (13) présente une rainure (97) de maintien, sensiblement en forme de V, orientée en biais vers le haut, destinée à un côté de la boucle de fil, la position inclinée de la rainure de maintien (97) étant orientée, au moment de la saisie de la boucle de fil, de manière sensiblement parallèle à celle-ci.

12. Machine à suture endoscopique selon la revendication 1 **caractérisée en ce que** la griffe (12) présente une contre-lame (106), dont la pointe (107) est orientée de manière sensiblement opposée à la pointe (105) de la lame principale (104).
